# EUROPEAN PATENT APPLICATION

(11) **EP 1 808 130 A1**
(43) Date of publication of application: **18.07.2007**
(21) Application number: 05785930.8
(22) Date of filing: 20.09.2005
(51) Int. Cl.: A61B 8/12

(54) **ULTRASONIC TRANSDUCER, ULTRASONIC ARRAY AND ULTRASONIC ENDOSCOPE SYSTEM**

(30) Priority: 24.09.2004 JP 2004277462; 30.09.2004 JP 2004286910
(71) Applicant: Olympus Corporation, Tokyo 151-0072 (JP)
(72) Inventor: WAKABAYASHI, Katsuhiro, 1920043 (JP); SAWADA, Yukihiko, 3420041 (JP); MIZUNUMA, Akiko, 1920065 (JP); IMAHASHI, Takuya, Kanagawa 2140006 (JP); SATO, Sunao, 1920032 (JP); FUJIMURA, Takanao, 2290001 (JP)
(74) Representative: von Hellfeld, Axel
(86) International application number: PCT/JP2005/017315
(87) International publication number: WO 2006/033331

(57) **Abstract**

An electronic radial type ultrasonic transducer arraying, in a cylindrical form, a plurality of ultrasonic transducer elements for transmitting and receiving ultrasound, and for internally storing cables corresponding to the respective ultrasonic transducer elements for transmitting a drive signal for driving each of the ultrasonic transducer elements, featuring a conductive film on a surface of an insulator through which the cable group is led, and also comprising an acoustic lens constituted of a lens part which is connected to a ground wire and featured as a convex form and a cover part covering an easily breakable part or conductor material which is electrically connected to a plurality of ultrasonic transducers.

## Description

### Technical Field

The present invention relates to an electronic radial scanning type ultrasonic transducer.

### Background Art

An electronic scanning type ultrasonic transducer is equipped on a part of an endoscope that is inserted into an abdomen, the use of which makes it possible to extract a clear image of a digestive canal wall and deep organs such as the pancreas, gall bladder, et cetera, in a good image quality without being negatively influenced by abdominal gasses or bone. The electronic scanning type ultrasonic transducer is constituted of no less than tens of elements and a number of coaxial cables for transmission and reception equivalent to the number of elements. When connecting an electrode of each element of the electronic scanning type ultrasonic transducer to a signal transmission/reception-use coaxial cable, a common method is to solder a core lead of the coaxial cable to a signal electrode of each element and solder a shield wire of the coaxial cable to a ground electrode of each element.

Among such electronic scanning type ultrasonic transducers, a convex type, linear type, and radial type have been utilized for an endoscope, as noted above. The radial type is for transmitting/receiving an ultrasonic beam in a circumferential direction and is categorized into a mechanical radial scanning system that transmits/receives an ultrasonic beam radially by rotating the transducers and an electronic radial scanning system that transmits/receives an ultrasonic beam radially by arraying a plurality of piezoelectric elements on the circumference of a cylindrical form and electronically controlling them (e.g., refer to a patent document 1).

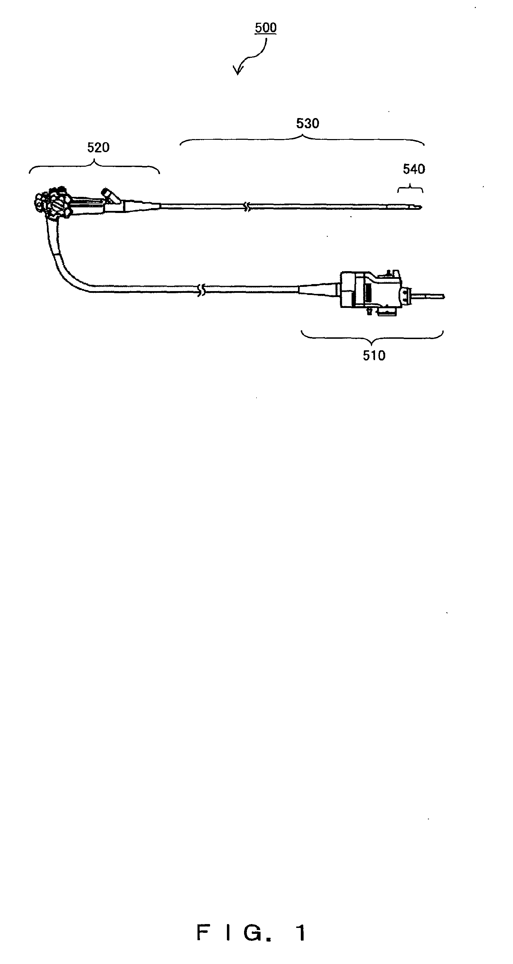

Fig. 1 is a diagram showing a conventional ultrasonic endoscope.

The ultrasonic endoscope 500 shown in Fig. 1 comprises a connection part 510, an operation part 520, and an insertion part 530 which comprises a head part 540.

The connection part 510 of the ultrasonic endoscope is connected to ultrasonic diagnostic equipment to constitute an ultrasonic endoscope system.

The operation part 520 performs curving operations of the insertion part 530 in the left, right, up and down directions and operation of the head part 540 (via user operation, for example).

The head part 540 is equipped with an ultrasonic transducer array constituted of a plurality of ultrasonic transducers being lined up continuously in addition to the ultra compact camera; a selected ultrasonic transducer from among the plurality thereof of the ultrasonic transducer array transmits or receives ultrasound. Further, the ultrasound received by the ultrasonic transducer array is converted into an electrical signal for displaying on the display or other such device as an image.

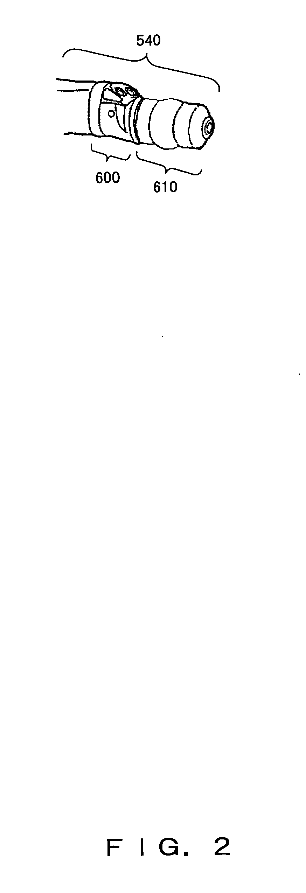

Fig. 2 is an enlarged diagram of the head part 540 shown in Fig. 1.

As shown in Fig. 2, the head part 540 comprises an ultrasonic transducer array 610 and a camera part 600 equipped with an ultra compact camera, an illumination device, and other such devices.

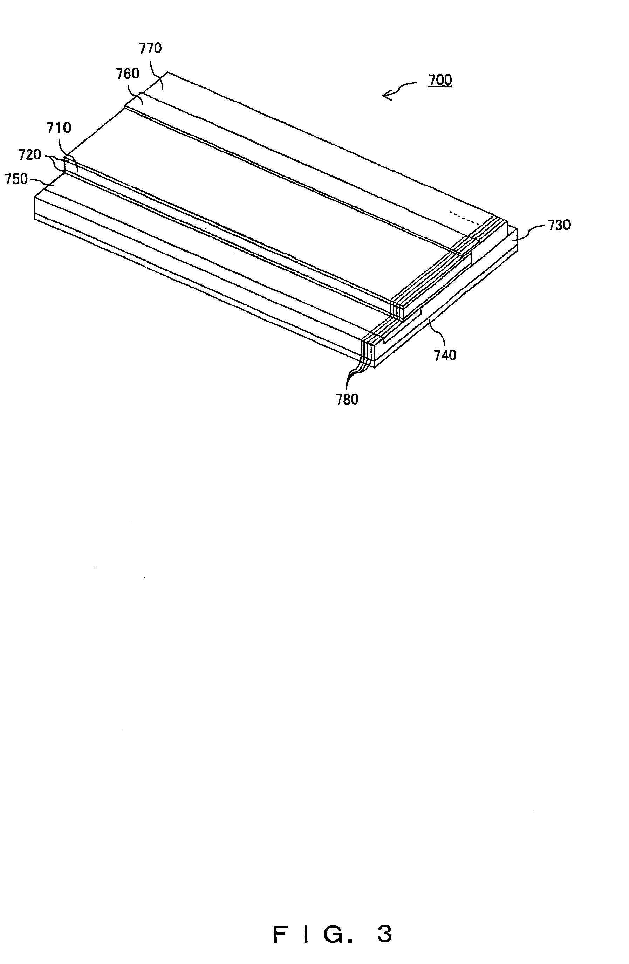

Fig. 3 is a diagram exemplifying an ultrasonic transducer array. Note that the ultrasonic transducer array shown in Fig. 3 shows a part of the ultrasonic transducer array 610 shown in Fig. 2.

The ultrasonic transducer array 700 shown in Fig. 3 comprises a piezoelectric element 710, an electrode layer 720, a first acoustic matching layer 730, a second acoustic matching layer 740, a conductive resin 750, a conductor material 760 and a board 770.

The piezoelectric element 710, electrode layer 720, first acoustic matching layer 730, second acoustic matching layer 740, conductive resin 750, conductor material 760 and board 770 are divided into a plurality of elements by commonly featured grooves 780, which results in the constitution of the plurality of ultrasonic transducers.

The end surfaces of the ultrasonic transducer array 700 that are perpendicular to the longitudinal direction of the ultrasonic transducer array 700 are connected with one another to form a cylindrical form, then an acoustic lens is equipped on the outer circumference of the cylindrically formed ultrasonic transducer array 700, thereby constructing the radial system (i.e., the electronic radial scanning system) ultrasonic transducer array for transmitting ultrasound in a circular pattern (e.g., refer to patent document 1 or 2).

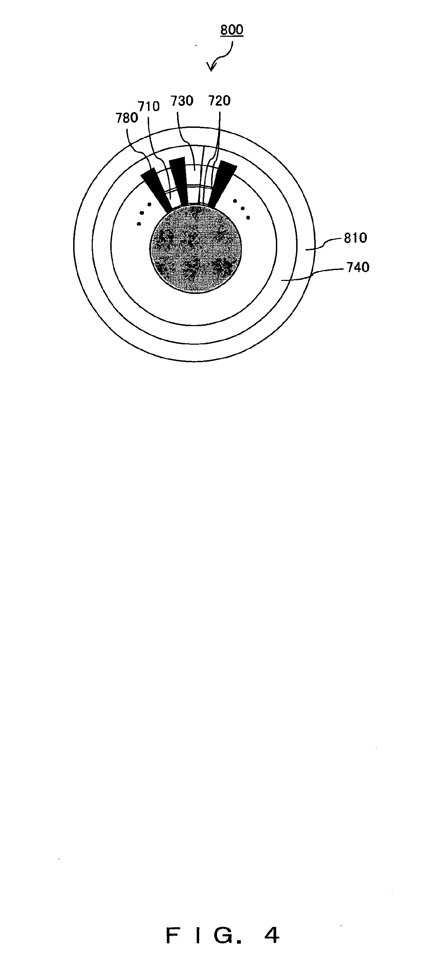

Fig. 4 is a diagram showing a cross section of a radial system ultrasonic transducer array when the ultrasonic transducer array 700 shown in Fig. 3 is structured to be a radial system ultrasonic transducer array.

The ultrasonic transducer array 800 shown in Fig. 4 is cylindrically formed in such a manner that the second acoustic matching layer 740, of the first and second acoustic matching layers 730 and 740, is placed on the outside, and that an acoustic lens 810 is equipped on the outer circumference of the second acoustic matching layer 740.

Ultrasound transmitted from the piezoelectric element 710 by way of the first and second acoustic matching layers 730 and 740 is converged to a prescribed focus by the acoustic lens 810.

Meanwhile, it is desirable for the head part of an ultrasonic endoscope to be reduced in diameter as much as possible, and for not only a radial system ultrasonic transducer array but also any ultrasonic transducer array mounted onto the ultrasonic endoscope to be configured to be as small as possible.

However, the configuration disclosed in patent document 1 forms a signal wiring between a cable and each ultrasonic transducer by using a flexible printed circuit (FPC) board and a connector, causing it be long and hard, thus creating problems such as the insertion of an ultrasonic endoscope equipped with the ultrasonic transducer causing pain to a patient receiving the insertion and a degraded operability of the apparatus. There has also been a problem of electrical safety concerning a human body if not all the signal wiring is connected to ground (GND) .

In addition, in an ultrasonic endoscope employing an electronic radial scanning system, size must be considered in order to be put into actual practice even though inventions are disclosed. That is, the diameter of the entirety of an ultrasonic transducer must be reduced in order to insert the ultrasonic transducer into a human body, and accordingly piezoelectric elements forming the ultrasonic transducer must inevitably be made smaller. There is also a need for arraying a plurality (e.g., approximately 200 pieces) of piezoelectric elements in directions encompassing a full 360 degrees, requiring that the size of each piezoelectric element be reduced to the order of 0.1 mm or less.

Under such a size limitation, miniaturization of an electronic radial system ultrasonic transducer is faced with various problems such as how to connect a long cable in the assembly of a transducer.

Although the cable is covered with an insulative material, a transducer must be produced that will not harm a patient even if a failure occurs, such as a live lead being exposied as a result of a part of the insulative covering of the cable being damaged, no matter how low the possibility of a failure may be. In order to eliminate a failure such as that described above, it is conceivable for example to insert a metallic pipe into the inside of an insulative material bundling a plurality of cables and connect the pipe to the ground. However, such a configuration is unsuitable to making the diameter of an ultrasonic transducer small in consideration of tolerance (i.e., the difference between the allowable maximum and minimum sizes in a machining process, for example).

An additional measure that can accomplish the miniaturization of an ultrasonic transducer array is having the piezoelectric element, acoustic matching layer or board of the ultrasonic transducer array be made to be thin (e.g., approximately 0.1 mm thick). If this is done, however, the array constituent components such as the piezoelectric element, acoustic matching layer or board will become easily breakable. Array constituent components such as the piezoelectric element and acoustic matching layer have been constantly reduced in thickness, keeping pace with a shift to higher frequencies of ultrasound for use in ultrasonic endoscopes in recent years.

As such, the piezoelectric element, acoustic matching layer or board, some of the constituent components of an ultrasonic transducer array mounted onto the ultrasonic endoscope, have been configured to be very easily broken, and there is accordingly a risk of breaking array constituent components such as the piezoelectric element and acoustic matching layer if, for example, the ultrasonic transducer array receives a shock. If an array constituent component such as piezoelectric element or acoustic matching layer is broken, there is a risk that a fragment of the array constituent component will fall off the ultrasonic endoscope.

The present invention accordingly aims at providing an ultrasonic transducer enabling a secure wire harnessing by fixing a cable by positioning it in relation to a transducer and achieving a small diameter with improved transducer safety.

The present invention also aims at providing an ultrasonic transducer array that will prevent a fragment of an array constituent component from falling off an ultrasonic endoscope even if the array constituent component, such as the piezoelectric element or acoustic matching layer, is broken as a result of receiving a shock.

Patent document 1: Japanese Registered Patent No. Sho 63-14623

Patent document 2: Laid-Open Japanese Patent Application Publication No. H05-42146

### Disclosure of Invention

In order to solve the problems described above, the present invention adopts the following comprisal.

An electronic radial type ultrasonic transducer according to the present invention is one arraying, in a cylindrical form, a plurality of ultrasonic transducer elements for transmitting and receiving ultrasound and internally storing cables corresponding to the respective ultrasonic transducer elements for transmitting a drive signal for driving each of the ultrasonic transducer elements, wherein a conductive film is featured on the surface of an insulator through which the cable group is led and the surface is connected to a ground wire.

A configuration may also be such that the ground wire is connected to the cable group by way of the conductive film.

A hole may be featured on a side face of the insulator and the other end of the ground wire, of which one end is connected to the cable group, may be led through the hole and adhered to the conductive film.

A surface of a flange part of the insulator may be equipped with a plurality of electrode pads which may connect to the cable corresponding to each electrode pad and also connect to the electrodes of the ultrasonic transducer elements by way of conductive wires.

The insulator may be featured with a flange on one end of an approximately cylindrical form.

The external surface and the internal surface of the approximately cylindrical part of the insulator may be featured with respective conductive films by means of plating.

The plating may be applied via a wet plating process.

The conductive film may be constituted of a plurality of metallic films.

The plurality of metallic films may be layered in the order of nickel, copper, nickel and gold.

The insulator may be constituted of engineering plastics.

The insulator may be constituted of polysulfone, polyether imide, polyphenylene oxide or epoxy resin.

The preferred thickness of the conductive film is between 1 and 50 micrometers. The ideal thickness of the conductive film is 20 micrometers.

The scope of the present invention encompasses an ultrasonic endoscope system comprising the above described electronic radial type ultrasonic transducer.

According to the present invention, the production method of an electronic radial type ultrasonic transducer comprises: a structure sheet production process for producing a structure sheet arraying a plurality of ultrasonic transducer elements for transmitting and receiving ultrasound; a cylinder forming process for forming the structure sheet into a cylindrical form; a circular plate equipment process for equipping the inside of an opening part of the cylindrically formed structure sheet with a circular plate; a plating process for forming conductive films respectively on the external surface and the internal surface of an insulator of which one end of an approximately cylindrical form is featured with a flange; a cable harnessing process for leading a plurality of cables through the insulator, adhering one end of the cables to the respective connectors of a plurality of electrode pads equipped on the flange surface of the insulator, and connecting a ground wire to the cable group by way of the conductive film; an insulator insertion process for inserting the insulator into the structure sheet until the flange of the insulator obtained by the cable harnessing process comes into contact with the circular plate of the structure sheet obtained by the circular plate equipment process; and a connection process for connecting, by conductive wires, the electrodes of the ultrasonic transducer elements to the electrode pads equipped on the flange surface of the insulator which is inserted in the insulator insertion process.

The plating, process may be carried out via a wet plating method.

The production method of an electronic radial type ultrasonic transducer may be such that a hole is featured in a side face of the insulator and the other end of the ground wire, of which one end is connected to the cable group, is led through the hole and adhered to the conductive film in the cable harnessing process.

Also according to the present invention, an ultrasonic transducer array comprises a plurality of ultrasonic transducers lined up continuously and an acoustic lens equipped on the outside of the plurality of ultrasonic transducers, and a selected ultrasonic transducer from among the plurality thereof transmits or receives ultrasound, wherein the acoustic lens, being constituted of an elastic material, comprises a lens part for converging ultrasound and a cover part for covering an easily breakable part of the ultrasonic transducer array.

The easily breakable part of the ultrasonic transducer array may be a piezoelectric element or acoustic matching layer.

Also according to the present invention, an ultrasonic transducer array comprises a plurality of ultrasonic transducers lined up continuously and an acoustic lens equipped on the outside of the plurality of ultrasonic transducers, and an electrically selected ultrasonic transducer from among the plurality thereof transmits or receives ultrasound, wherein the acoustic lens, being constituted of an insulative material, comprises a lens part for converging ultrasound and a cover part for covering a conductor material that is electrically connected to the plurality of ultrasonic transducers for selecting a plurality thereof.

The cover part of the ultrasonic transducer array may be configured to cover conductor materials not covered by a ground wire.

The conductor material of the ultrasonic transducer array may be a signal wire for transmitting a signal to the ultrasonic transducer or a board or wire for connecting the signal wire to the plurality of ultrasonic transducers.

The ultrasonic transducer array may be configured in such a manner that the plurality of ultrasonic transducers is structured to be lined up continuously in a circular pattern with an insertion axis at the center.

The ultrasonic transducer array may be configured in a manner such that the plurality of ultrasonic transducers is structured to be lined up continuously two-dimensionally.

The acoustic lens of the ultrasonic transducer array may be configured to include a siloxane linkage.

The acoustic lens of the ultrasonic transducer array may be configured to include iron oxide.

An ultrasonic endoscope system according to the present invention is one equipped with an ultrasonic transducer array comprising a plurality of ultrasonic transducers lined up continuously and an acoustic lens equipped on the outside of the plurality of ultrasonic transducers, and a selected ultrasonic transducer from among the plurality thereof transmits or receives ultrasound, wherein the acoustic lens, being constituted of an elastic material, comprises a lens part for converging ultrasound and a cover part for covering an easily breakable part of the ultrasonic transducer array.

An ultrasonic endoscope according to the present invention is one equipped with an ultrasonic transducer array comprising a plurality of ultrasonic transducers lined up continuously and an acoustic lens equipped on the outside of the plurality of ultrasonic transducers, and an electronically selected ultrasonic transducer from among the plurality thereof transmits or receives ultrasound, wherein the acoustic lens, being constituted of an insulative material, comprises a lens part for converging ultrasound and a cover part for covering a conductor material that is electronically connected to the plurality of ultrasonic transducers for selecting the ultrasonic transducer.

### Brief Description of Drawings

Fig. 1 is a diagram showing a conventional ultrasonic endoscope;
Fig. 2 is an enlarged diagram of the head part;
Fig. 3 is a diagram exemplifying an ultrasonic transducer array;
Fig. 4 is a diagram showing a cross section of a radial system ultrasonic transducer array when the ultrasonic transducer array shown in Fig. 3 is structured to a radial system ultrasonic transducer array;
Fig. 5 is a diagram showing an external configuration of an ultrasonic endoscope according to the present embodiment;
Fig. 6 is an enlarged diagram of a head part 3 of the ultrasonic endoscope 1 shown in Fig. 5;
Fig. 7 is a diagram showing a production process (part 1) of an ultrasonic transducer;
Fig. 8 is a diagram showing a production process (part 2) of an ultrasonic transducer;
Fig. 9 is a diagram showing a production process (part 3) of an ultrasonic transducer;
Fig. 10A is a diagram showing a production process (part 4) of an ultrasonic transducer;
Fig. 10B is a diagram showing a production process (part 4) of an ultrasonic transducer;
Fig. 10C is a diagram showing a production process (part 4) of an ultrasonic transducer;
Fig. 11 is a diagram showing a production process (part 5) of an ultrasonic transducer;
Fig. 12 is a diagram showing the surface of a flange 52 of a cylinder 50;
Fig. 13 is a diagonal view diagram of a cylinder 50;
Fig. 14A is a cross-directional view diagram of the production process shown in Fig. 11;
Fig. 14B is a cross-directional view diagram of the production process shown in Fig. 11;
Fig. 15A is a diagram showing a cylinder 50 featured with a hole in a cylinder part 53;
Fig. 15B is a diagram showing a cylinder 50 featured with a hole in a cylinder part 53;
Fig. 16A is a diagram exemplifying a pattern of plating on the cylinder part 53 of a cylinder 50;
Fig. 16B is a diagram exemplifying a pattern of plating on the cylinder part 53 of a cylinder 50;
Fig. 16C is a diagram exemplifying a pattern of plating on the cylinder part 53 of a cylinder 50;
Fig. 16D is a diagram exemplifying a pattern of plating on the cylinder part 53 of a cylinder 50;
Fig. 17A is a diagram exemplifying a pattern of connecting a cable 62 to a plated cylinder 50;
Fig. 17B is a diagram exemplifying a pattern of connecting a cable 62 to a plated cylinder 50;
Fig. 17C is a diagram exemplifying a pattern of connecting a cable 62 to a plated cylinder 50;
Fig. 17D is a diagram exemplifying a pattern of connecting a cable 62 to a plated cylinder 50;
Fig. 18 is a diagram showing a production process (part 6) of an ultrasonic transducer;
Fig. 19 is a diagram showing a cross-sectional diagram of Fig. 18;
Fig. 20 is a diagram showing a modified example of Fig. 19;
Fig. 21 is a diagram showing a cross section of an ultrasonic transducer array of a preferred embodiment of the present invention;
Fig. 22A is a diagram describing an assembly process of an ultrasonic transducer array;
Fig. 22B is a diagram describing an assembly process of an ultrasonic transducer array;
Fig. 23 is a diagram showing an ultrasonic transducer array in the state of an acoustic lens being assembled; and
Fig. 24 is a diagram showing a cross section of an ultrasonic transducer array of another preferred embodiment of the present invention.

### Best Mode for Carrying Out the Invention

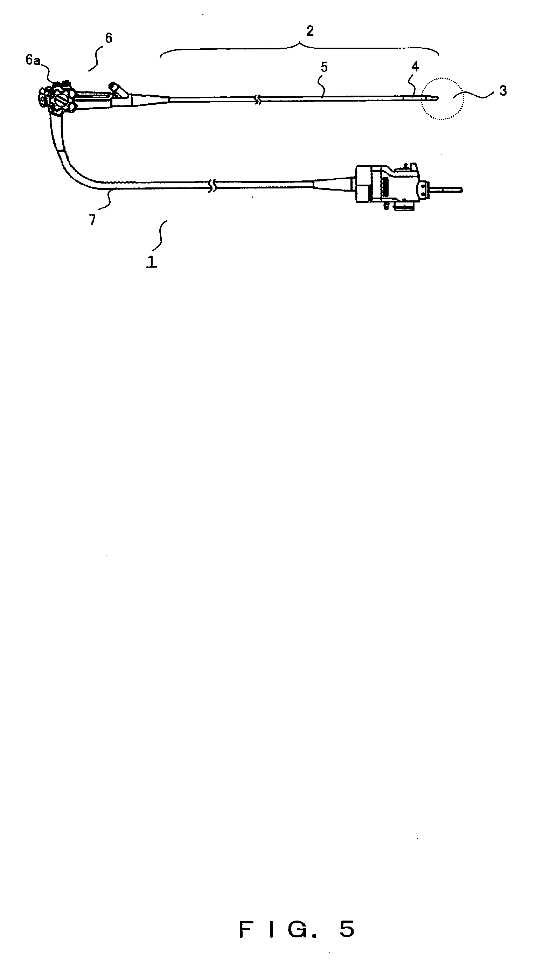

Fig. 5 shows an external configuration of an ultrasonic endoscope according to the present embodiment. The ultrasonic endoscope 1 comprises an operation part 6 at the base end of a slender insertion part 2. From a side part of the operation part 6 extends a universal cord 7 to be connected to a light source apparatus (not shown herein).

The insertion part 2 is constituted of connecting, in sequence starting at the head part, a head part 3, a bending part 4 that actuates bending and a flexible tube part 5 having flexibility. The operation part 6 is equipped with a curvature operation knob 6a and the bending part 4 can be bent by operating the curvature operation knob 6a.

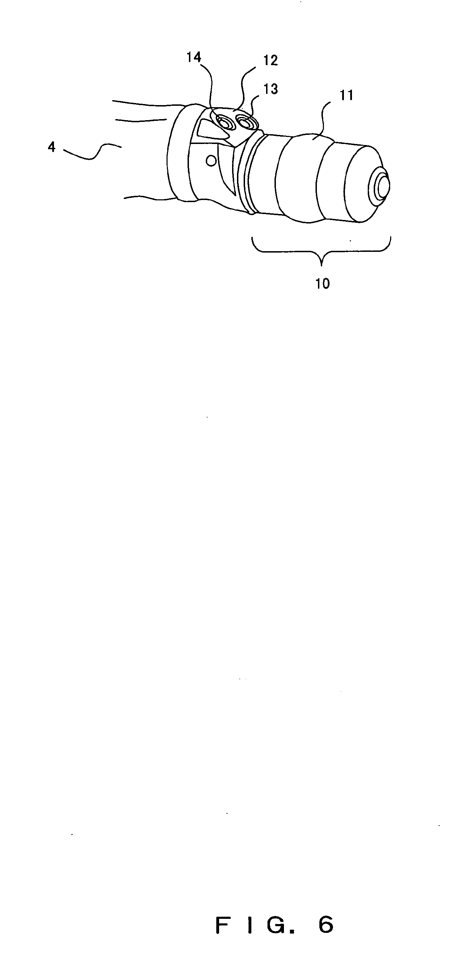

Fig. 6 is an enlarged diagram of the head part 3 of the ultrasonic endoscope 1 shown in Fig. 5. The head part 3 is equipped with an ultrasonic transducer 10 enabling an electronic radial type scan, and an inclination part 12 is equipped in between the bending part 4 and ultrasonic transducer 10. The ultrasonic transducer 10 is covered with a material forming an acoustic lens 11. The inclination part 12 is equipped with an illumination lens cover (not shown herein) constituting an illumination optical system for emitting an illumination light to an observation region, an observation-use lens cover 13 constituting an observation optical system for acquiring an optical image of an observation region and a forceps exit hole 14 that is an opening for projecting a treatment instrument.

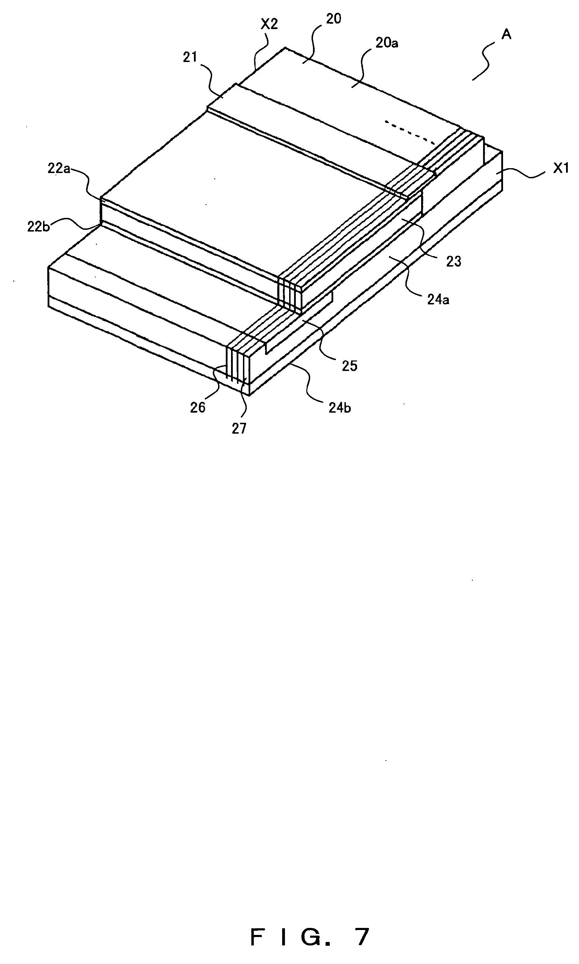

Next, a production process of the ultrasonic transducer 10 according to the present embodiment is described by using Figs. 7 through 19.

Fig. 7 is a diagram showing a production process (part 1) of an ultrasonic transducer. Referring to Fig. 7, the first step for forming the ultrasonic transducer 10 produces structure sheet A comprising a board 20, a conductor material 21, electrodes 22 (i.e., 22a and 22b), a piezoelectric element 23, acoustic matching layers 24 (i.e., first and second matching layers 24a and 24b), a conductive resin 25 and grooves (i.e., diced grooves) 26. To begin with, production of structure sheet A is described.

The second matching layer 24b is produced first, then the first matching layer 24a is produced. The grooves in the first matching layer 24a are featured by using a dicing saw (i.e., a high precision shearing machine) for example, followed by pouring conductive resin 25 into the grooves. Next a piezoelectric element 23 featured with electrodes 22a and 22b respectively on both of its opposing principal faces is attached, followed by the mounting of a board 20 on the side of the piezoelectric element 23. The surface of the board 20 is featured with an electrode layer 20a. Next the conductor material 21 for electrically connecting the electrode 20a to the electrode layer 22a is mounted.

Next, a plurality of grooves (i.e., diced grooves) 26 are produced by using a dicing saw for cutting into structure sheet A which is formed as described above. The desirable width of each of these grooves is between 20 to 50 micrometers. Note that the cut into structure sheet A should desirably be 20-50 micrometers into the thickness of only the second matching layer 24b. Approximately 200 of such grooves may be featured. The divided individual transducers are called ultrasonic transducer elements 27 hereinafter.

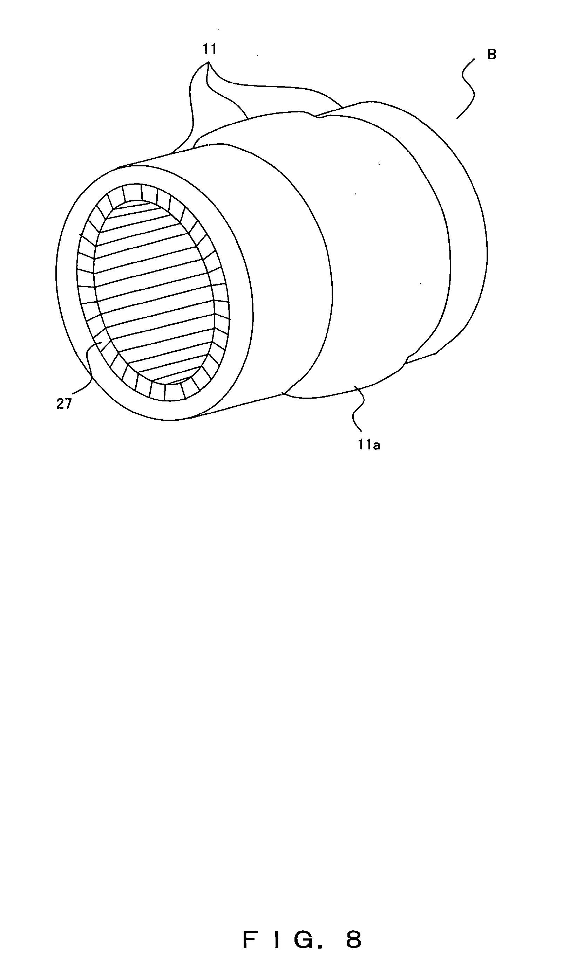

Next, structure sheet A is curved and formed into a cylindrical form in such a manner that side face X1 and side face X2 of the structure sheet touch each other, then an acoustic lens 11 (refer to Fig. 8) is formed on the circumference of the cylindrical form ("structure cylinder B" hereinafter) . As to the acoustic lens 11, it may be produced in advance as a single acoustic lens body and combined with the cylindrically formed structure sheet A, or it may be produced by placing the cylindrically formed structure sheet A into a mold followed by injecting an acoustic lens material thereinto. Note that the part actually functioning as an acoustic lens is lens part 11a of the acoustic lens 11.

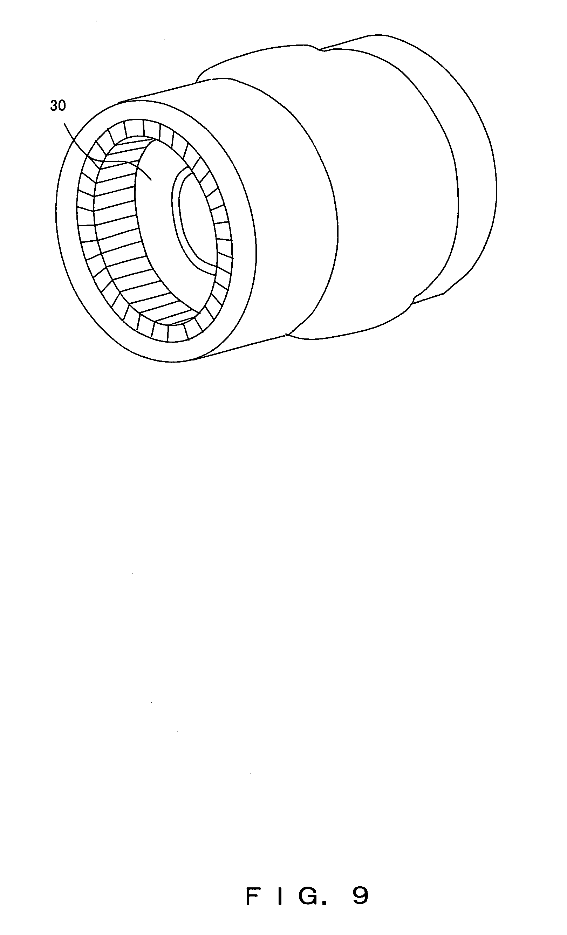

Next, circular plates 30 (i.e., 30a and 30b) are mounted on the inside of an opening part of structure cylinder B as shown in Figs. 9 and 10A. Circular plate 30a is mounted so as to be positioned on the board 20. Likewise, circular plate 30b is mounted onto an opening part on the other side. Circular plate 30b is mounted to be positioned on the conductive resin 25.

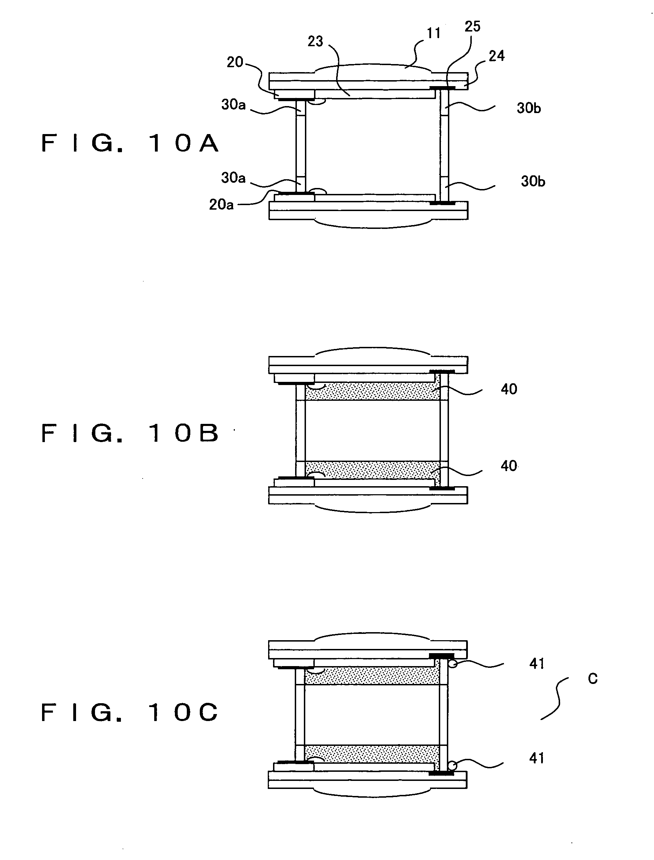

Fig. 10 shows a cross section of structure cylinder B with the circular plates 30 being mounted. Mounting of the circular plates 30 (i.e., 30a and 30b) (refer to Fig. 9 or 10A) is followed by filling of the space between circular plates 30a and 30b with a backing material 40 (refer to Fig. 10B). Next, a conductor material (i.e., a copper wire) 41 is mounted on the conductive resin 25 (refer to Fig. 10C) (the structure cylinder produced as shown in Fig. 10 is named "structure cylinder C" hereinafter).

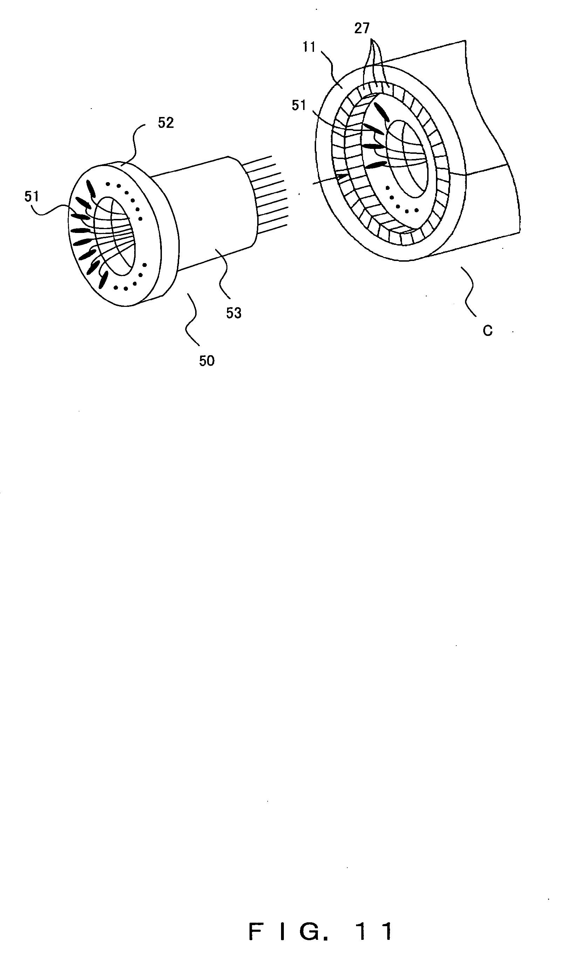

Next, a cylindrically formed cylinder 50 is inserted from one opening part side of structure cylinder C (i.e., the side equipped with the board 20) as shown in Fig. 11. The cylinder 50 is constituted of a cylinder part 53 and a circular flange 52 featured toward an end thereof.

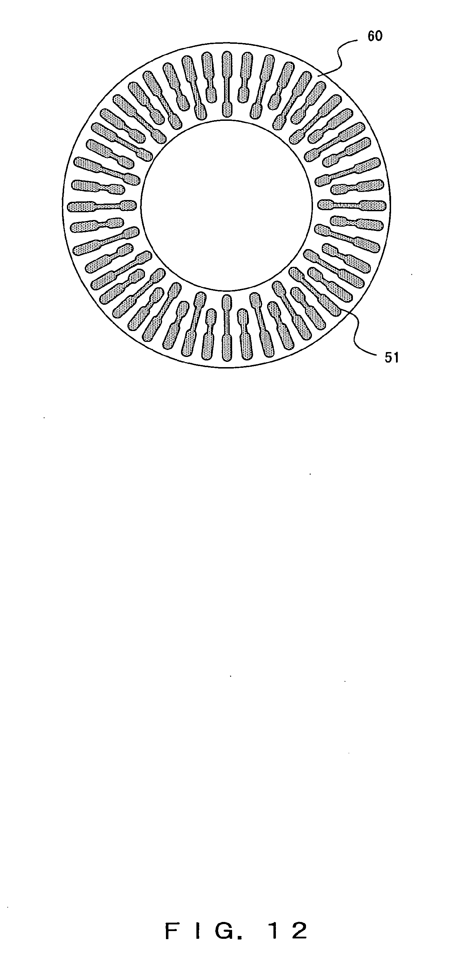

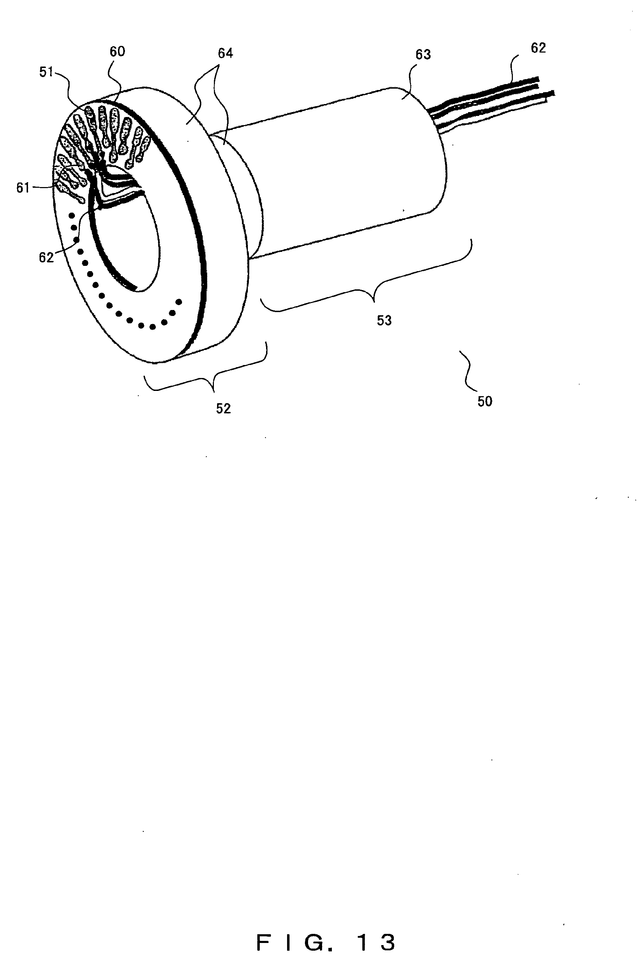

Fig. 12 shows a surface of the flange 52 of the cylinder 50 and Fig. 13 shows a diagonal view diagram of the cylinder 50. The surface of the flange 52 is equipped with a flexible printed circuit (FPC) board 60 on which the surface is equipped with electrode pads 51 numbering in the tens or hundreds. Furthermore, a cable bundle 62 is led internally through the cylinder 50 and the tips of each cable are soldered to each electrode pad 51; in other words, the cable 62 is connected by soldering 61 at the inside (i.e., toward the center of circle) of the electrode pad 51. Note that the cable 62 is usually a coaxial cable for noise reduction.

The cylinder 50 is made of an insulator material (e. g. , engineering plastics). The insulator material can be polysulfone, polyether imide, polyphenylene oxide, epoxy resin, or other such materials. The surface of the cylinder part 53 is plated with a conductor material.

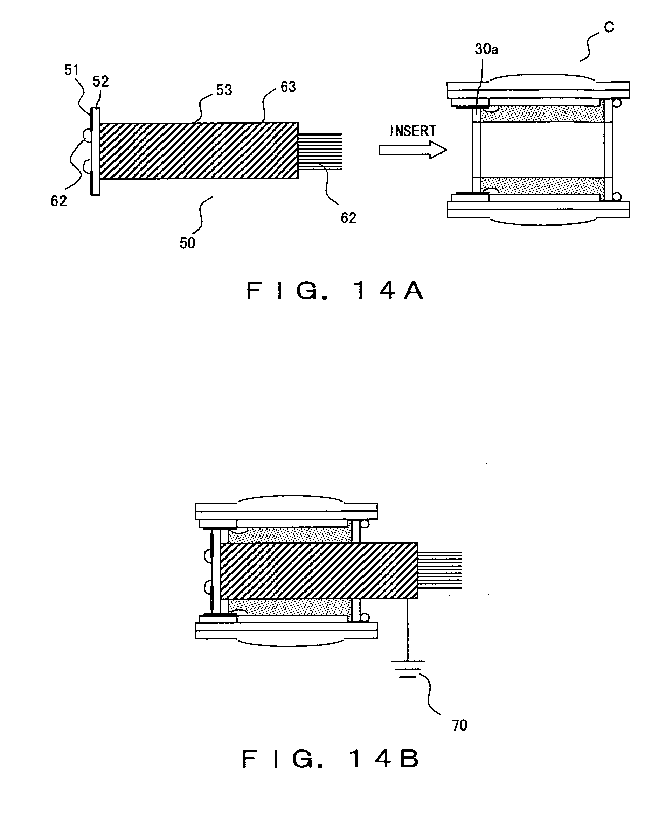

When inserting the cylinder 50 thus connected to the cable 62 into structure cylinder C (refer to Fig. 14A), the flange 52 part of the cylinder 50 hits the circular plates 30 of the structure cylinder C, fixing the position of the cylinder 50, thus positioning it on the inside of the ultrasonic transducer (refer to Fig. 14B) . When the cylinder 50 is inserted and positioned, it is connected to a GND 70 by way of the ground wire (GND).

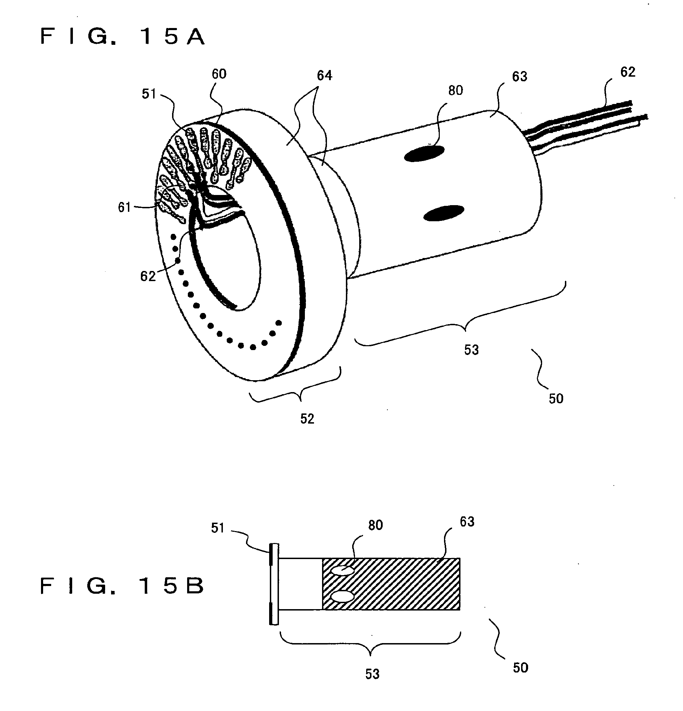

Note that a part of the cylinder part 53 of the cylinder 50 may be featured with one or a plurality of holes (i.e., a through hole(s) in the side face of the cylinder) 80 as shown in Fig. 15. This is described later.

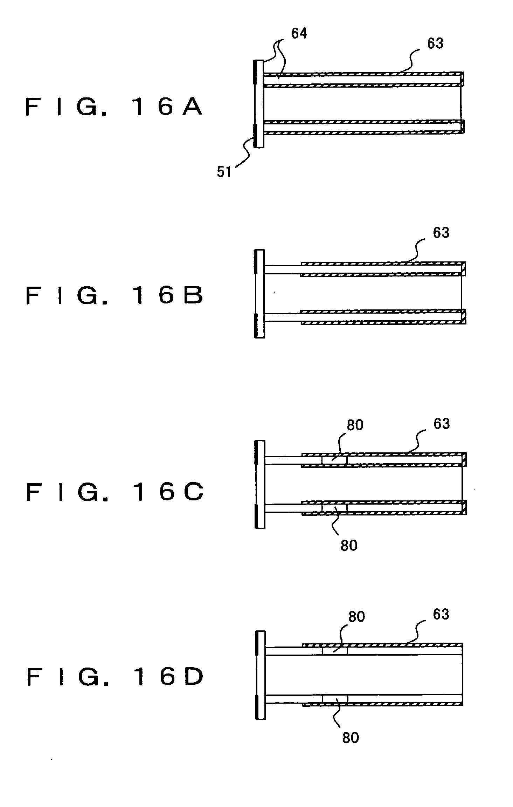

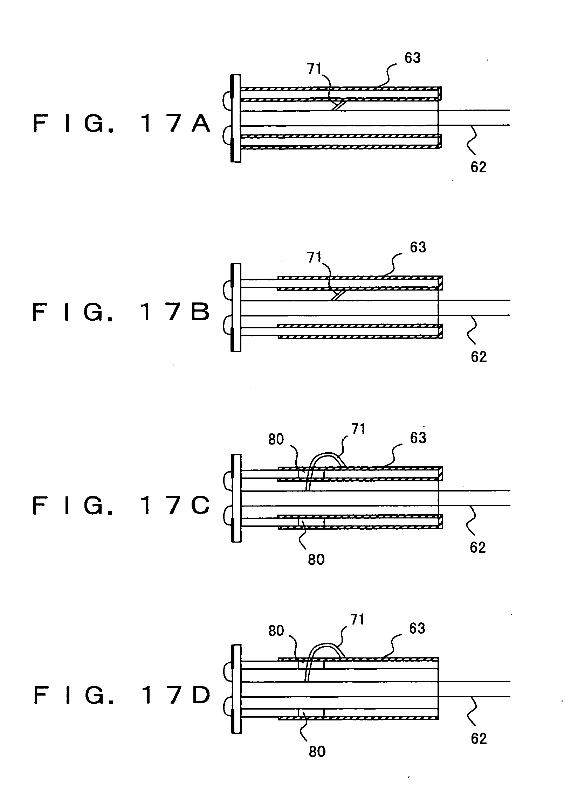

Now a further description of the cylinder 50 is provided by referring to Figs. 16 and 17.

Fig. 16 exemplifies a pattern of plating on the cylinder part 53 of a cylinder 50 after producing the cylinder 50 by using an insulative material 64. Fig. 16A shows a case in which a conductive film 63 is featured by plating the entirety of the external and internal surfaces of the cylinder part 53 with a conductor material. Fig. 16B shows a case in which a conductive film 63 is featured by plating a prescribed part of the external and internal surfaces of the cylinder part 53 with a conductor material. Fig. 16C shows a case in which a conductive film 63 is featured by plating a prescribed part of the external and internal surfaces of the cylinder part 53, which has been described in Fig. 15, with a conductor material. Fig. 16D shows a case in which a conductive film 63 is featured by plating a prescribed part of the external surface of the cylinder part 53, which has been described in Fig. 15, with a conductor material.

The plating method may be discretionary (e.g., sputtering, vapor deposition, etc.) provided that it is capable of forming a conductive film; the preferable method, however, is the formation of a film by a wet plating process such as electroplating or chemical plating (electroless plating). The reason is that wet plating is capable of easily forming a thicker film and plating on the internal and external circumferences of a cylinder, in contrast to sputtering, in which it is difficult to form a thick film and plate on the internal circumference of a cylinder. The thickness of the metallic film should be between 1 and 50 micrometers to effectively function as a conductive film, with approximately 20 micrometers being ideal. Films that are too thin do not function effectively as conductive films, and an adequate thickness is required in order to prevent peeling off when applying soldering. Alternatively, the surface of the cylinder part 53 of the cylinder 50 may be wrapped with a metal foil.

Note that a preferable plating method is to form films respectively on the internal and external surfaces of the insulative cylinder because the stress is well balanced in terms of thermal expansion and other such factors when this is done, thereby obtaining a film having no peeling off or cracking of the film even on a thin body. In this case the plating on the internal and external surfaces is preferably electrically connected to each other. That is, the plating is applied to the opening part of the cylinder, thus providing continuous plating from the internal surface to the external surface.

It is preferable that a metallic thin film formed by plating be a thin film of a plurality of metals in place of a single-layer film. The present embodiment is configured to apply the plating by starting with nickel (Ni), followed by copper (Cu), nickel (Ni) and gold (Au). These metals have respective roles here. In the first step, the cylinder part 53 of the cylinder 50 is plated with Ni as the base. In the next step, it is plated with Cu, which has good properties of conductivity, softness and extensibility. Next it is plated over with Ni, which has a good corrosion resistance. It is next plated over with Au to prevent the oxidization of Ni. Note that plating with a plurality of thin metallic films improves the strength of the plated insulative cylinder.

Fig. 17 exemplifies methods of connecting a cable 62 to a plated cylinder 50. Fig. 17A shows the case of connecting the cable 62 to the cylinder 50 shown in Fig. 16A. Fig. 17B shows the case of connecting the cable 62 to the cylinder 50 shown in Fig. 16B. Fig. 17C shows the case of connecting the cable 62 to the cylinder 50 shown in Fig. 16C. Fig. 17D shows the case of connecting the cable 62 to the cylinder 50 shown in Fig. 16D.

Referring to Figs. 17A, 17B, 17C and 17D, a ground wire 71 extends from the cable 62, and the ground wire 71 is connected to a conductive film 63 plated on the side surface of the cylinder part 53. When these are individually referred to, Figs. 17A and 17B show that the ground wire 71 is joined to the conductive film 63 on the internal surface side, and Figs. 17C and 17D show that the ground wire 71 is led through a hole 80 penetrating the side wall of the cylinder and joined to the conductive film 63 on the external surface side. The configurations shown in Figs. 17C and 17D allow the hole 80 to penetrate the side wall of the insulative cylinder and for the ground wire 71, which is very important in terms of electrical safety, to be led through nearby the ultrasonic transducer. Note that the position or number of the hole 80 is not particularly constrained.

The above is a description of the cylinder 50. Next, the process after the cylinder 50 is inserted and positioned will be described (refer to Fig. 14).

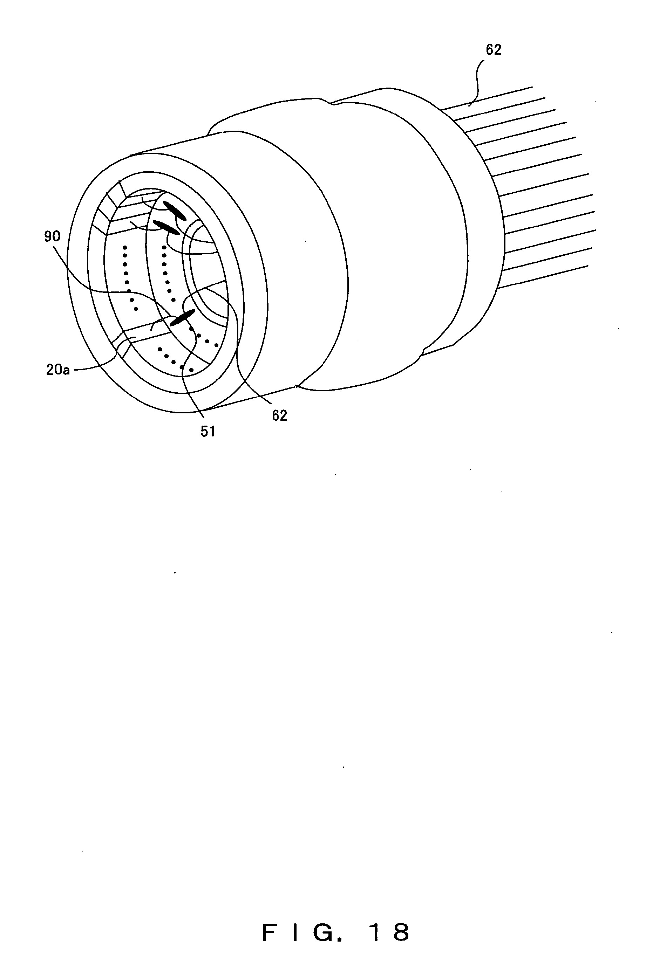

Fig. 18 shows external parts of electrode pads 51 (i.e., the electrode pad part toward the external circumference direction of the circle) being connected to respective electrodes 20a of the ultrasonic transducer elements 27 by using wires 90 after the cylinder 50 is inserted and positioned (refer to Fig. 14).

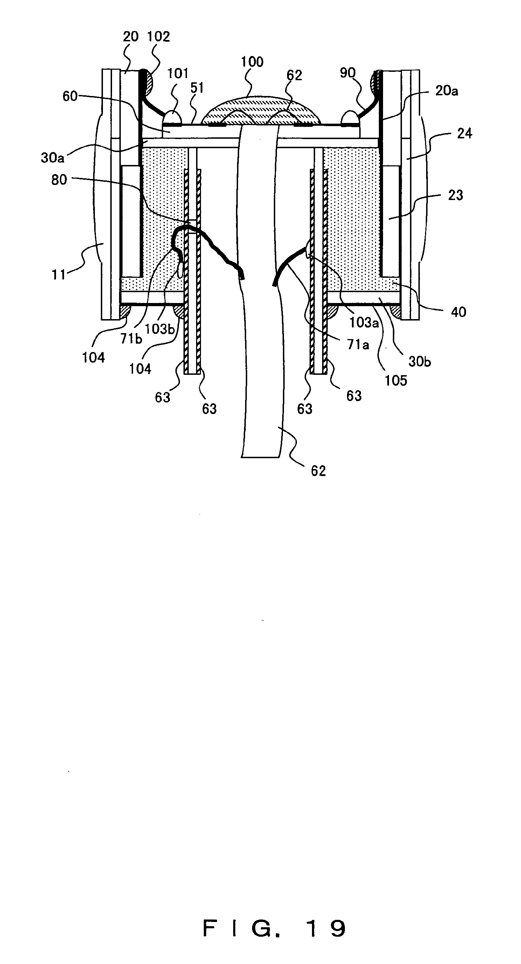

Fig. 19 shows a cross-sectional diagram of Fig. 18. As described above, the cable 62 is connected to the center direction side of the flange of the electrode pads 51 by soldering. One end of a wire 90 is connected to the external circumference direction side of the flange of the electrode pads 51 with soldering 101 and the other end is connected to a signal-side electrode 20a positioned on a board 20 of the transducer element by soldering 102. Note that the connection uses a short wire 90 so as to prevent the wire from contacting an adjacent signal-side electrode 20a. In addition, the entirety of the connection parts of the cable 62 and electrode pads 51 are covered with a potting resin 100 in order to prevent the cable 62 from falling off the electrode pads 51 as a result of the cable 62 being extended due to the application of a load thereto.

The surface of a circular plate 30b is covered with a film of copper foil 105, and the surface of a circular plates 30 is further connected to the cylindrical side surface of the acoustic matching layer 24 and cylinder 50 by a conductive resin (e.g., soldering) 104.

Note that the ground wires 71 (i.e., 71a and 71b) extended from the cable 62 are connected to the conductive film 63 by soldering 103a and 103b respectively as described for Fig. 17. Additionally, the conductive film 63 is grounded by a ground wire (not shown herein).

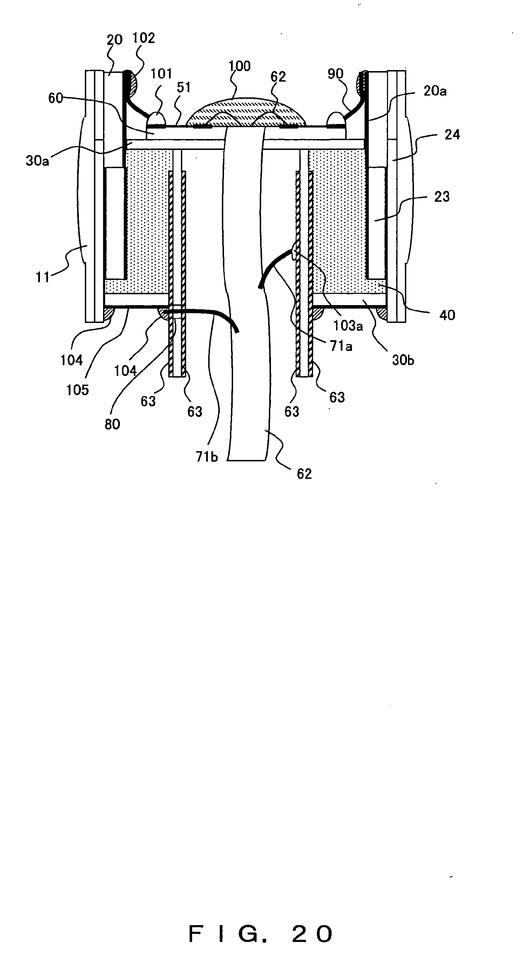

Fig. 20 shows a modified example of Fig. 19. Fig. 20 shows an example of the position of the hole 80 of the cylinder 50 being different from the configuration shown in Fig. 19, and of joining the ground wire 71b directly to the copper foil 105 featured on the circular plate 30b by using a conductive resin 104 such as solder. As such, the ground wire 71 may be separated into one wire (wire 71a in this case) to be joined to the conductive film on the cylinder 50 and another (wire 71b in this case) to be connected to the GND of the piezoelectric element (i.e. , connected to the copper foil on the structure cylinder) .

Note that the connection of the cable to the transducer element by using the cylinder as in the present embodiment can also be applied to an electronic radial type ultrasonic transducer employing a capacitive Micromachined Ultrasonic Transducer (c-MUT) in lieu of being limited to the ultrasonic transducer employing a piezoelectric element.

Thusly using the cylinder enables a process of a thin wall thickness by taking advantage of the characteristics of a cylindrical form. Also, the strength is higher than that of a square pipe or other shape because there is no biased stress. Also, the surface of an insulator constituting the cylinder is invested with a conductor material in the form of an adherence, improving electrical safety by being connected to the GND. It is also possible to pierce a hole in the side wall of the insulative cylinder and lead the ground wire, which is very important in terms of electrical safety, from the proximity of the transducer and connect it to a GND such as the piezoelectric element to begin with. This configuration brings forth the benefit of noise reduction as a result.

Also, when connecting a cable to the signal-side electrode 20a of the transducer element, the cable can be fixed by positioning it onto the transducer, and therefore it is possible to more easily and securely connect the cable than when connecting the cable directly to the signal-side electrode 20a.

Also, the durability of the connection part of the cable is improved against tension applied to the cable since the cable is connected to the electrode pad of the cylinder, then the electrode pad is connected to the signal-side electrode 20a by a wire instead of being connected to the cable directly thereto. The production process is also enabled to be divided into one process for connecting the cable to the cylinder and another process for inserting the cylinder connected to the cable into structure cylinder C and connecting it to the signal-side electrode 20a by way of a wire, making it possible to connect the electrode pad to the signal-side electrode 20a by using a short wire in the latter process, and therefore eliminating mistakes such as shorting or erroneous connection to a signal-side electrode 20a of another ultrasonic transducer element.

Also enabled is plating at a uniform thickness since there are no corners. The wet plating method is capable of covering the external and internal surfaces of the cylinder part of the cylinder with a metallic thin film, balancing the stress in terms of a thermal expansion and other such factors, thereby making it possible to obtain a film without causing detachment, cracking, et cetera of the film even on the thin wall. Also, structuring the metallic film to have a plurality of layers makes it possible to secure a prescribed film thickness in its entirety and a necessary electric current capacity.

As described above, the plating of the cylinder with a plurality of metallic films improves the adhesiveness of the metallic thin film to the insulative material. The plated metallic thin film is also capable of constituting a reinforcement member. Also, the forming of the metallic thin film thinner than the wall of a metal pipe makes it possible to reduce the diameter without the necessity of paying attention to tolerance, unlike the case of using a metal pipe. Meanwhile, the present invention eliminates biased stress, thus avoiding distortion; this contrasts to sputtering and other such methods, which apply a film only on one side and hence cause strain.

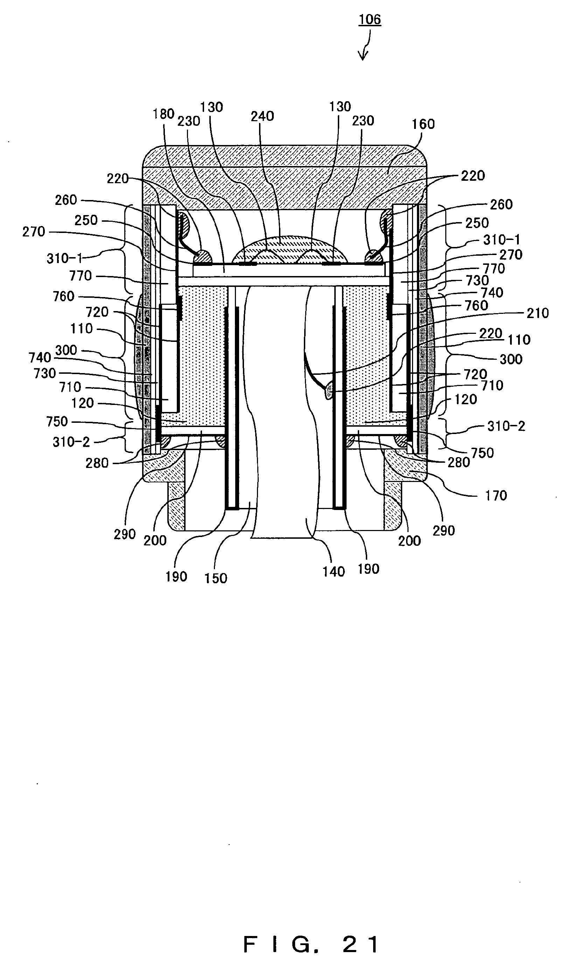

Fig. 21 is a diagram showing a cross section of an ultrasonic transducer array of a preferred embodiment of the present invention. Note that the same component sign is assigned to the same comprisal as the radial system ultrasonic transducer array 800 shown in Fig. 4.

The ultrasonic transducer array 106 (corresponding to the above described ultrasonic transducer array 10) shown in Fig. 21, being a radial system ultrasonic transducer array to be equipped on the head part 540 of the ultrasonic endoscope 500 shown in Fig. 1, comprises a piezoelectric element 710, electrode layers 720, a first acoustic matching layer 730, a second acoustic matching layer 740, a conductive resin 750, a conductor material 760, a board 770 and an acoustic lens 110. Note that a plurality of ultrasonic transducers (corresponding to the ultrasonic transducer elements 27) are configured by featuring a plurality of grooves commonly to the piezoelectric element 710, electrode layers 720, first acoustic matching layer 730, second acoustic matching layer 740, conductive resin 750, conductor material 760 and board 770.

The ultrasonic transducer array 106 comprises a backing material 120; a cylinder 150 for leading a cable 140, which is constituted of a bundle of individual signal wires 130 electrically connected respectively to a plurality of ultrasonic transducers, through the inside of the ultrasonic transducer array 106; a head plate 160 equipped on the head part of the ultrasonic transducer array 106; and a corrugated tube connection cylinder 170 equipped at the end part of the ultrasonic transducer array 106 for connecting an ultrasonic endoscope (e.g., the camera part 600 shown in Fig. 2) and the ultrasonic transducer array 106 together.

The ultrasonic transducer array 106 also comprises a board 180 equipped on the head part of the cylinder 150 for connecting the signal wire 130 electrically to the board 770, and a board 200 equipped on a rear part of the cylinder 150 for connecting a plated part 190 thereof electrically to the conductive resin 750. Note that the configuration is such that the plated part 190 is connected to a GND wire 210 (corresponding to ground wire 71), which is led through the inside of the cable 140 and extended to the cylinder 150 by means of soldering 220.

The respective ends of the individual signal wires 130 going through the cylinder 150 are connected to respectively prescribed pads 230, which are equipped on the board 180 and are fixed with a potting resin 240. The individual pads 230 are connected to respective prescribed pads 250 equipped on the board 180 by way of the wiring thereon. Also, the individual pads 250 are connected to prescribed respective wires 260 at one end of each wire by soldering 220, and the other end of each wire in the set of wires 260 is connected to respective prescribed conductor material 270 (corresponding to the electrode 20a) equipped on the board 770 by soldering 220.

With this setup, a signal is transmitted to the piezoelectric element 710 by way of the signal wire 130, wire 260, board 770 and one of the electrode layers 720 and ultrasound is transmitted from the piezoelectric element 710.

The plated part 190 is connected to a copper foil 290, which is featured on the board 200, by a conductive resin 280, and the copper foil 290 is connected to the conductive resin 750 by the conductive resin 280.

With this connection, the other electrode layers 720 are electrically connected to the GND.

The characteristic of the ultrasonic transducer array 106 lies where the acoustic lens 110 comprises a lens part 300 convexly formed for converging ultrasound transmitted from the piezoelectric element 710 at a prescribed focus, and cover parts 310 (i.e., 310-1 and 310-3) for covering easily breakable part of the piezoelectric element 710, first acoustic matching layer 730, second acoustic matching layer 740 and board 770, or for covering conductor materials electrically connected to a plurality of ultrasonic transducers, such as the signal wires 130, board 180, wires 260 and board 770, for electrically selecting a plurality of ultrasonic transducers. Incidentally, the configuration is such that the end part of the cover part 310-1 on the head part side of the ultrasonic transducer array 106 is joined to the head plate 160 with an adhesive or other such joining method. Also, the end part of the cover part 310-2 on the rear end part side of the ultrasonic transducer array 106 is joined to the corrugated tube connection cylinder 170 with an adhesive or other such joining method.

In other words, the cover part 310-1 covers the easily breakable parts (e.g., the piezoelectric element 710, first acoustic matching layer 730, second acoustic matching layer 740, and board 770) and also the conductor materials (e.g., the signal wires 130, board 180, wires 260 and board 770) which are located between the center axis and outer circumference of the ultrasonic transducer array 106 and are not covered with a GND wire. The cover part 310-2 mainly covers the easily breakable parts (e.g., the piezoelectric element 710, first acoustic matching layer 730 and second acoustic matching layer 740).

As for the ultrasonic transducer array 106, the piezoelectric element 710 is constructed of ceramics; the first acoustic matching layer 730 is constructed of an epoxy resin containing a filler such as alumina (i.e., aluminum oxide) or titania (i.e., titanium dioxide); the second acoustic matching layer 740 is constructed of an epoxy resin not containing a filler; the board 770 is constructed of a glass epoxy resin or other such substance; and the backing material 120 is constructed of a gelatinous epoxy resin containing a filler such as alumina.

In addition, for the ultrasonic transducer array 106 with three-layer matching, the configuration may be such that the first acoustic matching layer is constructed of machinable ceramics or carbon containing a filler or fibers; the second acoustic matching layer is constructed of an epoxy resin containing a small amount of filler such as alumina, titania, or other such substance; and the third acoustic matching layer is constructed of an epoxy resin not containing a filler.

In addition, for the ultrasonic transducer array 106, the configuration is such that the acoustic lens 110 is constructed of an elastic material and also an insulative material (e.g., a silicone resin containing a siloxane linkage). Note that the material for structuring the acoustic lens 110 may be discretionary provided that it is durable against shock and is insulative (for example, a fluorine-containing elastomer) in lieu of being limited.

As described above, it is possible to contain a fragment within the ultrasonic transducer array 106 by virtue of the acoustic lens 110 even if the ultrasonic transducer array 106 is given a shock and an easily breakable part is broken because the easily breakable part of the piezoelectric element 710, first acoustic matching layer 730, second acoustic matching layer 740 and board 770 are covered by the cover parts 310 of the acoustic lens 110, which is constructed of an elastic material. This configuration makes it possible to prevent a fragment of the easily breakable part s from falling off the ultrasonic endoscope even if some easily breakable part s are broken as a result of the ultrasonic transducer array 106 being given a shock.

Also, the conductor materials, e. g. , the signal wires 130, board 180, wires 260 and board 770, are covered by the cover parts 310 of the acoustic lens 110, which are constructed of an insulative material, and therefore an excess voltage will not be applied externally to the ultrasonic transducer array 106 due to the presence of the acoustic lens 110 even if the excess voltage is applied to a conductor material for any reason. This configuration is capable of preventing an excess voltage from being applied externally to the ultrasonic endoscope even if the excess voltage is applied to a conductor material for any reason.

Next is a description of the assembly process of the ultrasonic transducer array 106.

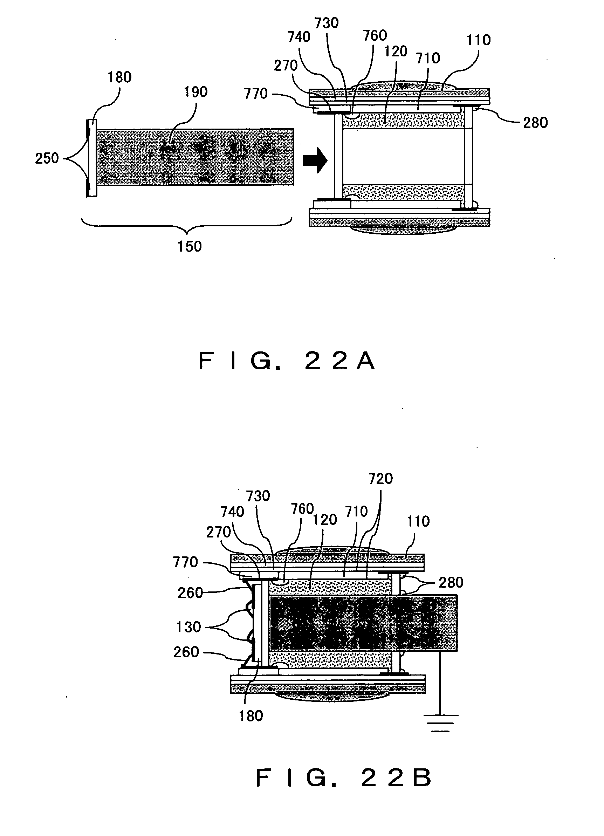

Figs. 22A and 22B are each diagrams for describing the assembly process of the ultrasonic transducer array 106. Note that the same component sign is assigned to the same configuration as the one shown in Fig. 21.

In the first step, a plate-formed ultrasonic transducer array is constructed as in the case of the ultrasonic transducer array 700 shown in Fig. 3, then a cylindrical form is formed by mutually connecting, perpendicular to the longitudinal direction thereof, each end surface of the plate-formed ultrasonic transducer array .

Next, the internal circumference of the cylindrically formed ultrasonic transducer array is equipped with the backing material 120 and the outer circumference of the cylindrically formed ultrasonic transducer array is equipped with the acoustic lens 110.

Next, the cylinder 150 invested with the cable 140 is inserted into the inside of the cylindrically formed ultrasonic transducer array as shown in Fig. 22A.

Next, the signal wire 130 is electrically connected to the electrode layer 720 on the center side of the cylinder 150 of the piezoelectric element 710 by way of the conductor material 760, conductor material parts 270 and wires 260, and also the GND is electrically connected to the electrode layer 720 on the outside of the cylinder 150 of the piezoelectric element 710 by way of the conductive resin 750, conductive resin 280, copper foil 290 and plated part 190, as shown in Fig. 22B.

Next, the head plate 160 is joined with an adhesive or other such joining method to the head part of the cylindrically formed ultrasonic transducer array and the corrugated tube connection cylinder 170 is joined with an adhesive or other such method to the rear end part of the cylindrically formed ultrasonic transducer array, thereby structuring the ultrasonic transducer array 106 (refer to Fig. 21).

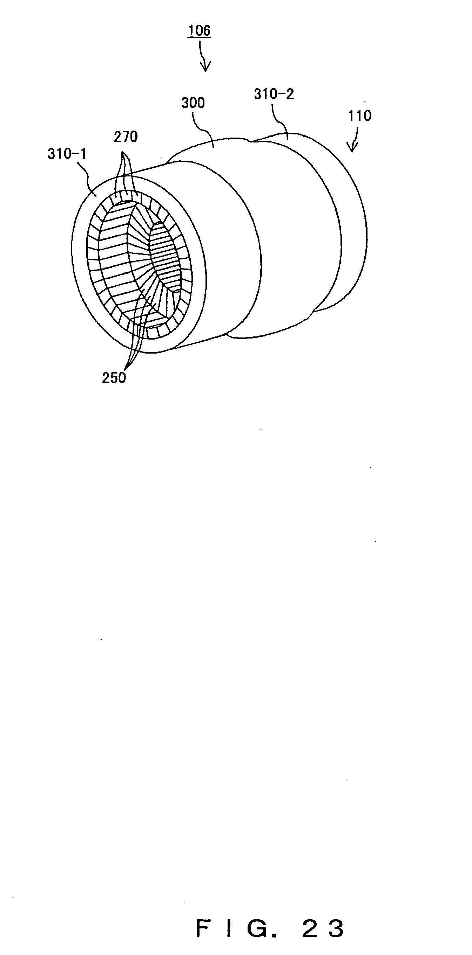

Fig. 23 shows a diagonal view diagram of the ultrasonic transducer array 106 in the state of the acoustic lens 110 being assembled. Note that the same component signs are assigned to the corresponding components of Fig. 21.

As shown in Fig. 23, the cover part 310-1 of the acoustic lens 110 covers the individual pads 250 featured on the board 180, the individual conductor material parts 270 featured on the board 770, and other such parts.

### <Other embodiments>

The present invention conceivably allows various comprisals in lieu of being limited to the embodiments as described above. Example comprisals include the following.

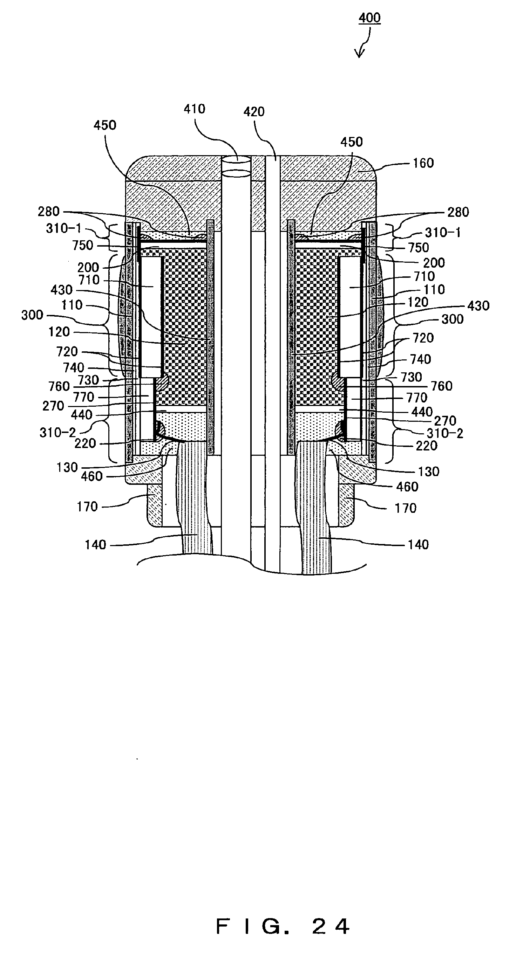

Fig. 24 is a diagram showing a cross section of an ultrasonic transducer array of another preferred embodiment of the present invention. Note that the same component sign is assigned to the corresponding components of Fig. 21.

The ultrasonic transducer array 400 shown in Fig. 24 is a so-called direct viewing type ultrasonic transducer array, and internally comprises a protection tube 430 (which is constructed of a metallic tube and also functions as a dam when filling with a backing material 120) equipping, on the inside of the ultrasonic transducer array 400, an optical system 410 (e.g., an ultra compact camera, illuminating device, or other such device equipped in the camera part 600 shown in Fig. 2) such as an ultra compact camera, illuminating device, or other such device, and an air/water supply hole 420. Note that a circular plate 440, shown in Fig. 24, also functions as a dam when filling with the backing material 120. The configuration also includes a head plate 160 and a board 200 with a filling of a conductive resin 450 between them. Also, a corrugated tube connection cylinder 170 and the circular plate 440 have a filling of a shield material 460 between them.

Also, the ultrasonic transducer array 400 shown in Fig. 24 is configured to electrically connect a signal wire 130 to the electrode layer 720 on the center side of the protection tube 430 of a piezoelectric element 710 situated at a rear part of the ultrasonic transducer array 400, that is, in the neighborhood of the corrugated tube connection cylinder 170.

As in the case of the ultrasonic transducer array 106 shown in Fig. 21, the characteristic of the ultrasonic transducer array 400 shown in Fig. 24 lies where the cover parts 310 of acoustic lens 110 cover easily breakable parts such as the piezoelectric element 710, first acoustic matching layer 730, second acoustic matching layer 740 and board 770, or conductor materials electrically connected to a plurality of ultrasonic transducers, such as signal wires 130, board 180, wires 260 and board 770, for electrically selecting a plurality of ultrasonic transducers. The configuration is also such that the end part of the cover part 310-1 is joined to the head plate 160 with an adhesive or other such joining method. Also, the end part of the cover part 310-2 is joined to the corrugated tube connection cylinder 170 with an adhesive or other such joining method.

In other words, cover part 310-1 mainly covers the easily breakable parts (e.g., the piezoelectric element 710, first acoustic matching layer 730 and second acoustic matching layer 740), while cover part 310-2 covers the easily breakable parts (e.g., the piezoelectric element 710, first acoustic matching layer 730, second acoustic matching layer 740 and board 770) and also the conductor materials (e.g., the signal wires 130, board 180, wires 260 and board 770) which are located between the center axis and outer circumference of the ultrasonic transducer array 106, and which are not shielded by a GND wire.

With this coverage, it is possible to contain a fragment within the ultrasonic transducer array 400 even if the ultrasonic transducer array 400 is given a shock and an easily breakable part is broken, and therefore it is possible to prevent the fragment from falling off an ultrasonic endoscope, as in the case of the ultrasonic transducer array 106 shown in Fig. 21.

This configuration is capable of preventing an excess voltage from being applied externally to the ultrasonic endoscope even if the excess voltage is applied to a conductor material because the excess voltage is never applied externally to the ultrasonic transducer array 400.

Note that a plurality of ultrasonic transducers according to the above-described embodiment are configured to be lined up continuously in one dimension as a result of a plurality of grooves being featured, in the plate-formed piezoelectric elements 710, in the direction perpendicular to the longitudinal direction thereof; the plurality of ultrasonic transducers, however, may also be configured in a manner to be lined up continuously in two dimensions by featuring a plurality of grooves, in the plate-formed piezoelectric elements 710, both in the longitudinal direction of the piezoelectric elements 710 and in the direction perpendicular thereto. Also, a radial system ultrasonic transducer array may be constructed by circularly lining up a plurality of ultrasonic transducers, which are configured to be lined up in two dimensions, with an insertion axis as the center.

The acoustic lens 110 according to the above- described embodiment may be configured to contain iron oxide (e.g., colcothar). Such a configuration of the acoustic lens 110 containing iron oxide is empirically known to improve the voltage resistance thereof.

The lens part 300 of the acoustic lens 110 according to the above described embodiment is configured to be convex; the form of the lens part 300, however, is discretionary, and can be concave.

The above-described embodiment is configured in a manner such that the cover parts 310-1 and 310-2 of the ultrasonic transducer array 106 (or, the ultrasonic transducer array 400) are mutually different in length in accordance with the structure of the ultrasonic transducer array 106 (or, the ultrasonic transducer array 400) ; the respective lengths of the cover parts 310-1 and 310-2 in the insertion direction, however, may be configured to be mutually the same when the size of places that the cover parts 310-1 and 310-2 respectively cover are the same.

The above-described embodiment may be configured in a manner such that the lens part 300 and cover parts 310 are different parts. That is, a cover part for covering easily breakable parts and conductor materials not covered by a GND wire may be equipped on the outer circumference of the ultrasonic transducer array 106 while the acoustic lens 110 only comprises the lens part 300. Incidentally, the cover part is to be constituted of an elastic and insulative material such as silicone resin.

As described above, the present invention is contrived to enable secure wiring by positioning and fixing a cable to a transducer, an improvement of electrical safety of the transducer and, furthermore, an improvement in diameter (the resulting diameter is smaller) thereof.

The present invention is also contrived to cover the easily breakable parts with the acoustic lens constructed of an elastic material, and therefore, even if the ultrasonic transducer array is given a shock and some easily breakable part is broken, the broken fragment can be contained within the ultrasonic transducer array. This configuration makes it possible to prevent the broken fragment from falling off the ultrasonic endoscope even if the ultrasonic transducer array is given a shock and some easily breakable part is broken.

The present invention is also contrived to cover conductor materials with the acoustic lens, which is constructed of an insulative material, thereby preventing an excess voltage from being applied externally to the ultrasonic transducer array if the excess voltage is applied to the conductor materials. This configuration prevents an excess voltage from being applied externally to the ultrasonic endoscope even if the excess voltage is applied to the conductor materials.

## Claims

1. An electronic radial type ultrasonic transducer arraying, in a cylindrical form, a plurality of ultrasonic transducer elements for transmitting and receiving ultrasound, and internally stored cables corresponding to the respective ultrasonic transducer elements for transmitting a drive signal for driving each of the ultrasonic transducer elements, wherein
a conductive film is featured on a surface of an insulator through which the cable group is led and the surface is connected to a ground wire.

2. The electronic, radial type ultrasonic transducer according to claim 1, wherein
the ground wire is connected to the cable group by way of the conductive film.

3. The electronic radial type ultrasonic transducer according to claim 1, wherein
a hole is featured on a side face of the insulator and the other end of the ground wire, of which one end is connected to the cable group, is led through the hole and then adhered to the conductive film.

4. The electronic radial type ultrasonic transducer according to claim 1, wherein
a surface of a flange part of the insulator is equipped with a plurality of electrode pads and connects to the cable corresponding to each electrode pad and also connects to the electrodes of the ultrasonic transducer elements by way of conductive wires.

5. The electronic radial type ultrasonic transducer according to claim 1, wherein
the insulator is featured with a flange on one end of an approximately cylindrical form.

6. The electronic radial type ultrasonic transducer according to claim 5, wherein
an external surface and an internal surface of the approximately cylindrical form part of the insulator are featured with respective conductive films by means of a plating process.

7. The electronic radial type ultrasonic transducer according to claim 6, wherein
the plating process is a wet plating process.

8. The electronic radial type ultrasonic transducer according to claim 1, wherein
the conductive film is constituted of a plurality of metallic films.

9. The electronic radial type ultrasonic transducer according to claim 8, wherein
the plurality of metallic films are layered in the order of, from the bottom layer, nickel, copper, nickel and gold.

10. The electronic radial type ultrasonic transducer according to claim 1, wherein
the insulator is constituted of engineering plastics.

11. The electronic radial type ultrasonic transducer according to claim 1, wherein
the insulator is constituted of polysulfone, polyether imide, polyphenylene oxide or epoxy resin.

12. The electronic radial type ultrasonic transducer according to claim 1, wherein
the thickness of the conductive film is between 1 and 50 micrometers.

13. The electronic radial type ultrasonic transducer according to claim 1, wherein
the thickness of the conductive film is 20 micrometers.

14. An ultrasonic endoscope system comprising an electronic radial type ultrasonic transducer according to claim 1.

15. A production method of an electronic radial type ultrasonic transducer, comprising:
a structure sheet production process for producing a structure sheet arraying a plurality of ultrasonic transducer elements for transmitting and receiving ultrasound;
a cylinder forming process for forming the structure sheet into a cylindrical form;
a circular plate equipment process for equipping the inside of an opening part of the cylindrically formed structure sheet with a circular plate;
a plating process for forming, by plating, conductive films respectively on an external surface and an internal surface of an insulator, of which one end of an approximately cylindrical form is featured with a flange;
a cable harnessing process for leading a plurality of cables through the insulator, adhering one end of the cables to the respective electrode pads of a plurality of electrode pads equipped on the flange surface of the insulator, and connecting a ground wire to the cable group by way of the conductive film;
an insulator insertion process for inserting the insulator into the structure sheet until the flange of the insulator obtained by the cable harnessing process comes into contact with the circular plate of the structure sheet obtained by the circular plate equipment process; and
a connection process for connecting, by conductive wires, the electrodes of the ultrasonic transducer elements to the electrode pads equipped on the flange surface of the insulator that is inserted into the insulator insertion process.

16. The production method of an electronic radial type ultrasonic transducer according to claim 15, wherein
the plating process is carried out by a wet plating method.

17. The production method of an electronic radial type ultrasonic transducer according to claim 15, wherein
a hole is featured in a side face of the insulator and the other end of the ground wire, of which one end is connected to the cable group, is led through the hole and then adhered to the conductive film in the cable harnessing process.

18. An ultrasonic transducer array comprising a plurality of ultrasonic transducers lined up continuously, in which a selected ultrasonic transducer from among the plurality thereof transmits or receives ultrasound, and an acoustic lens equipped on the outside of the plurality of ultrasonic transducers, wherein
the acoustic lens, being constituted of an elastic material, comprises a lens part for converging ultrasound and a cover part for covering an easily breakable part of the ultrasonic transducer array.

19. The ultrasonic transducer array according to claim 18, wherein
the easily breakable part is a piezoelectric element or acoustic matching layer.

20. An ultrasonic transducer array comprising a plurality of ultrasonic transducers lined up continuously, in which and an electrically selected ultrasonic transducer from among the plurality thereof transmits or receives ultrasound, and an acoustic lens equipped on the outside of the plurality of ultrasonic transducers, wherein
the acoustic lens, being constituted of an insulative material, comprises a lens part for converging ultrasound and a cover part for covering a conductor material that is electrically connected to the plurality of ultrasonic transducers for selecting a plurality thereof.

21. The ultrasonic transducer array according to claim 20, wherein
the cover part covers the conductor material not covered by a ground wire.

22. The ultrasonic transducer array according to claim 20, wherein
the conductor material is a signal wire for transmitting a signal to the ultrasonic transducer, or a board or wire for connecting the signal wire to the plurality of ultrasonic transducers.

23. The ultrasonic transducer array according to claim 18, wherein
the plurality of ultrasonic transducers is structured to be lined up continuously in a circular pattern with an insertion axis at the center.

24. The ultrasonic transducer array according to claims 18, wherein
the plurality of ultrasonic transducers is structured to be lined up continuously two-dimensionally.

25. An ultrasonic endoscope equipped with an ultrasonic transducer array comprising a plurality of ultrasonic transducers lined up continuously, in which a selected ultrasonic transducer from among the plurality thereof transmits or receives ultrasound, and an acoustic lens equipped on the outside of the plurality of ultrasonic transducers, wherein
the acoustic lens, being constituted of an elastic material, comprises a lens part for converging ultrasound and a cover part for covering an easily breakable part of the ultrasonic transducer array.

26. An ultrasonic endoscope equipped with an ultrasonic transducer array comprising a plurality of ultrasonic transducers lined up continuously, in which an electrically selected ultrasonic transducer from among the plurality thereof transmits or receives ultrasound, and an acoustic lens equipped on the outside of the plurality of ultrasonic transducers, wherein
the acoustic lens, being constituted of an insulative material, comprises a lens part for converging ultrasound and a cover part for covering a conductor material that is electrically connected to the plurality of ultrasonic transducers for selecting the ultrasonic transducer.
